# EUROPEAN PATENT APPLICATION

(11) **EP 4 755 370 A1**
(43) Date of publication of application: **10.06.2026**
(21) Application number: 25219832.0
(22) Date of filing: 01.12.2025
(51) Int. Cl.: A61K 9/00, A61K 9/20, A61K 31/407

(54) **PROCESS FOR PREPARATION OF KETOROLAC TROMETHAMINE SUBLINGUAL TABLET AND TABLET COMPOSITION THEREFOR**

(30) Priority: 04.12.2024 IN 202411095806
(71) Applicant: Athena Pharmaceutiques SAS, 78430 Louveciennes (FR)
(72) Inventor: CHAUDHARI, Mahendra Baliram, 400602 Maharashtra (IN); CHANDWANI, Omprakash Doulatram, 421301 Maharashtra (IN); NEHETE, Nitin Pandharinath, 421201 Maharashtra (IN); CHAUDHARI, Amol Yuvraj, 421202 Maharashtra (IN); SHAHANE, Anita Kunal, 421301 Maharashtra (IN)
(74) Representative: Grünecker Patent- und Rechtsanwälte PartG mbB

(57) **Abstract**

The present disclosure relates to a process for preparation of ketorolac tromethamine sublingual tablet and tablet composition therefor. The process comprises dry mixing ketorolac tromethamine, a diluent, and an antistatic agent to form a dry mix blend; intermediate blending a cushioning agent, a sweetener and a disintegrating agent to form an intermediate blend; drug blending the dry mix blend and the intermediate blend to form a drug blend; preparing a lubricated drug blend comprising sifting a lubricant, and lubricating the drug blend with the lubricant to form a lubricated drug blend; and compressing the lubricated drug blend to form a ketorolac tromethamine sublingual tablet.

## Description

### FIELD OF THE PRESENT DISCLOSURE

The present disclosure generally relates to a process for preparation of sublingual tablet composition. In particular, the present disclosure relates to a process for preparation of ketorolac tromethamine sublingual tablet and tablet composition therefor.

### BACKGROUND

Ketorolac tromethamine is tromethamine salt of ketorolac (C₁₅H₁₃NO₃. C₄H₁₁NO), with molecular formula C₁₉H₂₄N₂O₆ is also known by the chemical name "benzoyl-2,3-dihydro-1H-pyrrolizine-1-carboxylic acid". IUPAC name of ketorolac tromethamine is "2-amino-2-(hydroxymethyl)propane-1,3-diol;5-benzoyl-2,3-dihydro-1H-pyrrolizine-1-carboxylic acid".

Chemical structure of ketorolac tromethamine is represented below:

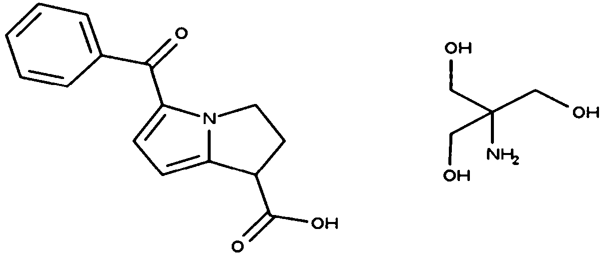

Ketorolac tromethamine is a synthetic pyrrolizine carboxylic acid derivative with anti-inflammatory, analgesic and antipyretic properties. Ketorolac tromethamine, a non-selective inhibitor of the cyclooxygenases (COX), inhibits both COX-1 and COX-2 enzymes.

Ketorolac tromethamine as an active ingredient exhibits a strong bitter taste and thus there is a need of efficient taste-masking to improve its palatability.

Ketorolac tromethamine available in the form of traditional tablet gets absorbed by the liver. In the liver, the drug is converted to metabolites before it is excreted in the bile. From bile the drug enters the digestive tract, from there, drugs are either eliminated in faeces or reabsorbed into the bloodstream. This way a major portion of the active drug is wasted. Additionally, the prevalent process for preparation has a problem of unequal distribution of the ketorolac tromethamine in the composition.

Accordingly, there is a need for a dosage form for ketorolac tromethamine which has quick dispersion with good mouth feel. Specifically, a dosage form with good organoleptic properties and positive effect on taste masking and mouth sensation. The need further includes a dosage form by which ketorolac tromethamine is absorbed directly and circulated through blood with minimum wastage of ketorolac tromethamine.

The need further includes a composition with such pharmaceutical excipients that promotes good organoleptic properties with positive effect on taste masking and mouth sensation; quick disintegration time in mouth; suitable flowability of drug blend; and convenience during tableting. Moreover, it is needed that the shortcoming of unequal distribution of the ketorolac tromethamine is minimized or removed.

### SUMMARY

To achieve the foregoing and other objects and needs, the present disclosure provides a process for preparation of ketorolac tromethamine sublingual tablet and ketorolac tromethamine sublingual tablet therefor. The ketorolac tromethamine sublingual tablet developed by the inventor is a sublingual tablet that has quick dispersion with good mouth feel. Further, the present disclosure provides a ketorolac tromethamine sublingual tablet in which the drug directly reaches the systemic circulation using blood vessels. This way the wastage of drug herein ketorolac tromethamine is minimized.

The present disclosure provides a process whereby efficient taste-masking is opted to improve its palatability of the drug considering the bitter taste of ketorolac tromethamine. The pharmaceutical excipients are selected within the specific range of percentage by weight for contributing into a tablet composition for sublingual or buccal administration to achieve quick dispersion with good mouth feel for the tablet.

The pharmaceutical excipients of the present ketorolac tromethamine sublingual tablet composition contribute towards good organoleptic properties with positive effect on taste masking and mouth sensation, quick disintegration time in mouth, suitable flowability of drug blend, and enhancing convenience during tableting. Moreover, the selection of pharmaceutical excipients exhibits no punch or die wall sticking during compression. Additionally, pharmaceutical excipients with similar particle size are used for minimizing the risk of unequal distribution of the ketorolac tromethamine.

In an embodiment, the present disclosure relates to a process for preparation of ketorolac tromethamine sublingual tablet. The process comprises dry mixing ketorolac tromethamine, a diluent, and an antistatic agent to form a dry mix blend; intermediate blending a cushioning agent, a sweetener and a disintegrating agent to form an intermediate blend; drug blending the dry mix blend and the intermediate blend to form a drug blend; preparing a lubricated drug blend comprising sifting a lubricant, and lubricating the drug blend with the lubricant to form a lubricated drug blend; and compressing the lubricated drug blend to form a ketorolac tromethamine sublingual tablet.

In one or more embodiments, in the step of dry mixing, the diluent is lactose monohydrate, and the diluent is present in an amount of about 55 to about 60 percent by weight; and the antistatic agent is colloidal anhydrous silica, and the antistatic agent is present in an amount of about 0.2 to about 0.8 percent by weight.

In one or more embodiments, in the step of intermediate blending, the cushioning agent is microcrystalline cellulose, and the cushioning agent is present in an amount of about 15 to about 20 percent by weight; the sweetener is sucralose, and the sweetener is present in an amount of about 3 to about 5 percent by weight; and the disintegrating agent is crospovidone, and the disintegrating agent is present in an amount of about 2 to about 7 percent by weight.

In one or more embodiments, in the step of lubricating the drug blend, the lubricant is magnesium stearate, and the lubricant is present in an amount of about 1 to about 2 percent by weight.

In one or more embodiments, the lactose monohydrate and microcrystalline cellulose are in a molar ratio of 3:1.

In one or more embodiments, the particle size of lactose monohydrate is not more than 150 microns.

In one or more embodiments, the ketorolac tromethamine sublingual tablet is packed in a blister package.

In another embodiment, the present disclosure relates to a ketorolac tromethamine sublingual tablet. The ketorolac tromethamine sublingual tablet comprises about 10 to about 15 percent by weight of ketorolac tromethamine; about 55 to about 60 percent by weight of a diluent; about 0.2 to about 0.8 percent by weight of an antistatic agent; about 15 to about 20 percent by weight of a cushioning agent; about 3 to about 5 percent by weight of a sweetener; about 2 to about 7 percent by weight of a disintegrating agent; and about 1 to about 2 percent by weight of a lubricant.

In one or more embodiments, the dosage amount of the ketorolac tromethamine sublingual tablet is 10 mg per tablet.

In one or more embodiments, the diluent is lactose monohydrate; the antistatic agent is colloidal anhydrous silica; the cushioning agent is microcrystalline cellulose; the sweetener is sucralose; the disintegrating agent is crospovidone; and the lubricant is magnesium stearate.

In one or more embodiments, the lactose monohydrate and microcrystalline cellulose are in the molar ratio of 3:1.

In one or more embodiments, the particle size of lactose monohydrate is not more than 150 microns.

In an embodiment, the friability is less than 1.5 %; the disintegration time is less than 300 seconds; and the hardness is from about 20 N to about 60 N.

### BRIEF DESCRIPTION OF THE FIGURES

The advantages and features of the present disclosure will become better understood with reference to the following detailed description and claims taken in conjunction with the accompanying drawing, in which:
FIG. 1 illustrates a process flow for preparation of a ketorolac tromethamine sublingual tablet, in accordance with an embodiment of the present disclosure.
FIG. 2 illustrates process details of dry mixing of ketorolac tromethamine, a diluent, and an antistatic agent, in accordance with an embodiment of the present disclosure.
FIG. 3 illustrates process details of intermediate blending of a cushioning agent, a sweetener and a disintegrating agent, in accordance with an embodiment of the present disclosure.
FIG. 4 illustrates details of drug blending the dry mix blend and the intermediate blend, in accordance with an embodiment of the present disclosure.
FIG. 5 illustrates details of lubrication, in accordance with an embodiment of the present disclosure.
FIG. 6 illustrates details of compression, in accordance with an embodiment of the present disclosure.
FIG. 7 illustrates details of packing of ketorolac tromethamine sublingual tablet, in accordance with an embodiment of the present disclosure.
FIG. 8A illustrates a graphical representation of the linear plot for the mean plasma concentration versus time for ketorolac tromethamine of test product and reference product, in accordance with an embodiment of the present disclosure.
FIG. 8B illustrates a graphical representation of semilogarithmic plot for the mean plasma concentration versus time for ketorolac tromethamine of test product and reference product, in accordance with an embodiment of the present disclosure.

### DETAILED DESCRIPTION

The exemplary embodiments described herein detail for illustrative purposes are subject to many variations in structure and design. It should be emphasized, however, that the present disclosure is not limited to a process for preparation of ketorolac tromethamine sublingual tablet and tablet composition therefor, as shown and described. It is understood that various omissions and substitutions of equivalents are contemplated as circumstances may suggest or render expedient, but these are intended to cover the application or implementation without departing from the spirit or scope of the claims of the present disclosure. Also, it is to be understood that the phraseology and terminology used herein is for the purpose of description and should not be regarded as limiting.

The use of terms "including," "comprising," or "having" and variations thereof herein is meant to encompass the items listed thereafter and equivalents thereof as well as additional items.

Further, the terms, "a" and "an" herein do not denote a limitation of quantity, but rather denote the presence of at least one of the referenced items.

As used herein, the term "about" will be understood by persons of ordinary skill in the art and will vary to some extent depending upon the context in which it is used. If there are uses of the term which are not clear to persons of ordinary skill in the art, given the context in which it is used, "about" will mean up to plus or minus 10 % of the particular term.

The present disclosure provides a process for preparation of ketorolac tromethamine sublingual tablet and tablet composition therefor. To achieve the foregoing and other objects and needs, the present disclosure provides a process for preparation of ketorolac tromethamine sublingual tablet and tablet therefor. The ketorolac tromethamine sublingual tablet developed by the inventor is a sublingual tablet that has quick dispersion with good mouth feel. Further, the present disclosure provides a ketorolac tromethamine sublingual tablet in which the drug directly reaches the systemic circulation using blood vessels. This way the wastage of drug herein ketorolac tromethamine is minimized.

The present disclosure provides a process whereby efficient taste-masking is opted to improve the palatability of the drug considering the bitter taste of ketorolac tromethamine. The pharmaceutical excipients are selected within the specific range of percentage by weight for contributing into a tablet composition for sublingual or buccal administration to achieve quick dispersion with good mouth feel for the tablet.

The pharmaceutical excipients contribute towards good organoleptic properties with positive effect on taste masking and mouth sensation; quick disintegration time in mouth; suitable flowability of drug blend; and convenience during tableting. Moreover, the selection of pharmaceutical excipients exhibits no punch or die wall sticking during compression. Additionally, pharmaceutical excipients with similar particle size for minimizing the risk of unequal distribution of the ketorolac tromethamine.

In one embodiment, the process for preparation of ketorolac tromethamine sublingual tablet is disclosed. Herein, the "ketorolac tromethamine sublingual tablet" and "ketorolac tromethamine ST" have been interchangeably used hereinafter without any limitations. The process for preparation of ketorolac tromethamine sublingual tablet comprises dry mixing ketorolac tromethamine, a diluent, and an antistatic agent to form dry mix blend; intermediate blending a cushioning agent, a sweetener and a disintegrating agent to form an intermediate blend; drug blending the dry mix blend and the intermediate blend to form a drug blend; preparing a lubricated drug blend comprising sifting a lubricant, and lubricating the drug blend with the lubricant to form a lubricated drug blend; and compressing the lubricated drug blend to form a ketorolac tromethamine sublingual tablet.

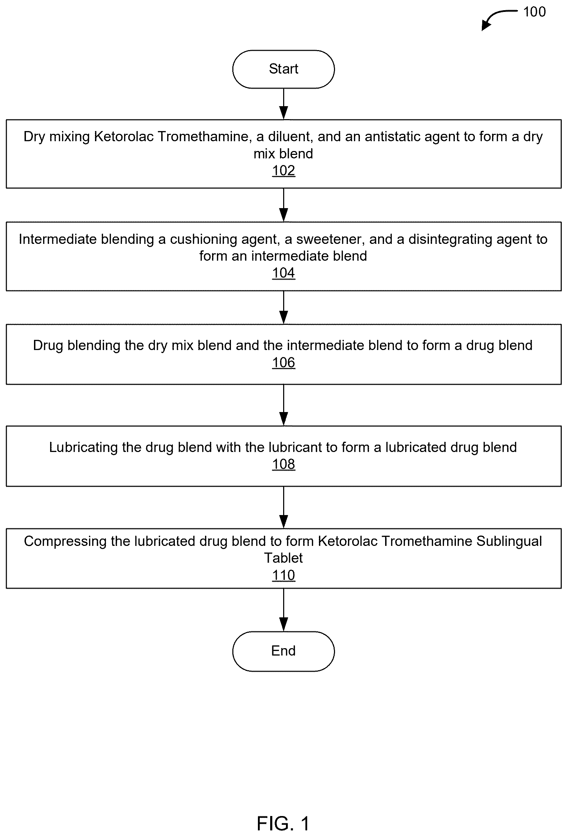

Referring to FIG. 1, the process 100 relates to a process for preparation of ketorolac tromethamine sublingual tablet. At step 102, the process 100 comprises dry mixing of ketorolac tromethamine, a diluent, and an antistatic agent to form dry mix blend. At step 104, the process 100 comprises intermediate blending a cushioning agent, a sweetener and a disintegrating agent to form an intermediate blend. At step 106, the process 100 comprises drug blending the dry mix blend and the intermediate blend to form a drug blend. At step 108, the process 100 comprises preparing a lubricated drug blend comprising sifting a lubricant and lubricating the drug blend with the lubricant to form a lubricated drug blend. At step 110, the process 100 comprises compressing the lubricated drug blend to form a ketorolac tromethamine sublingual tablet.

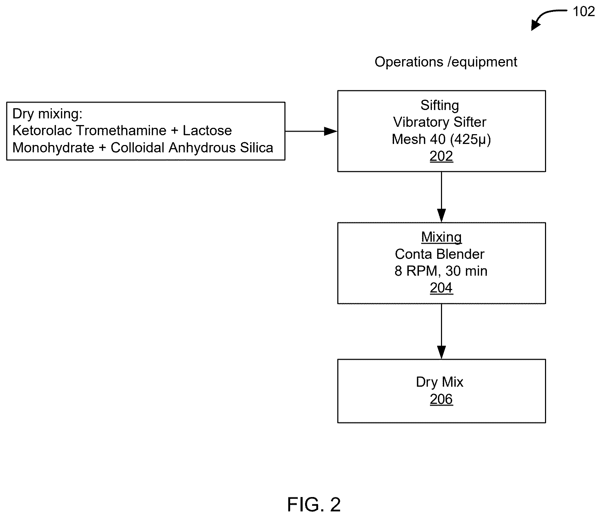

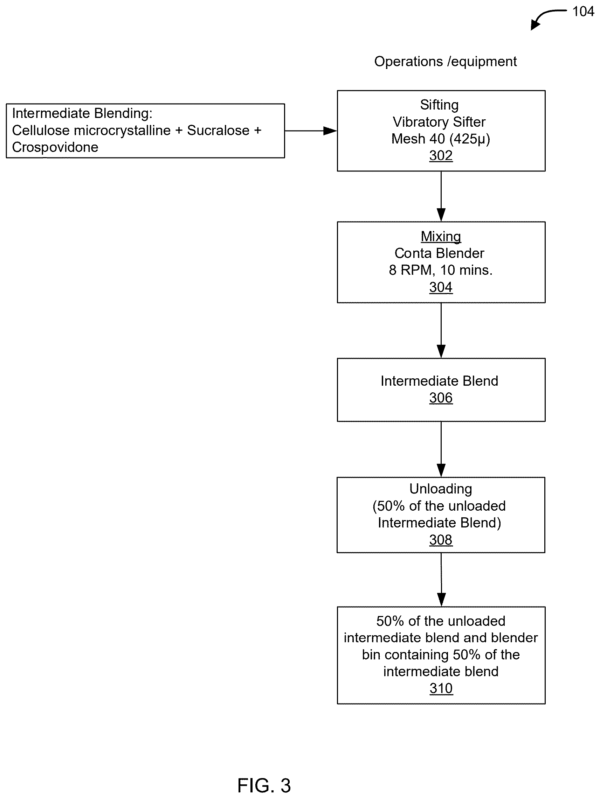

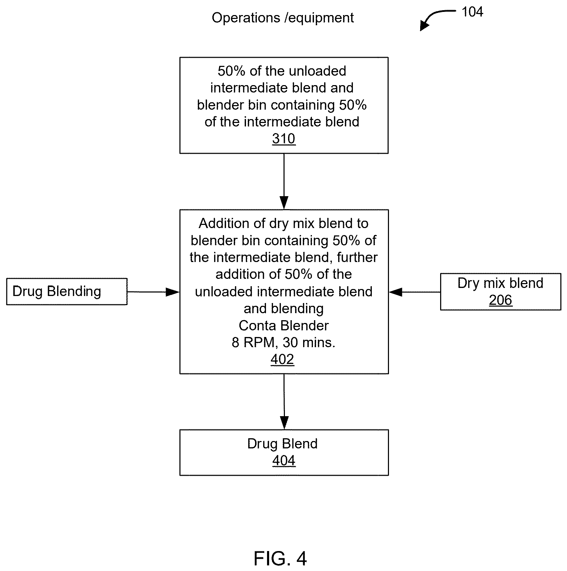

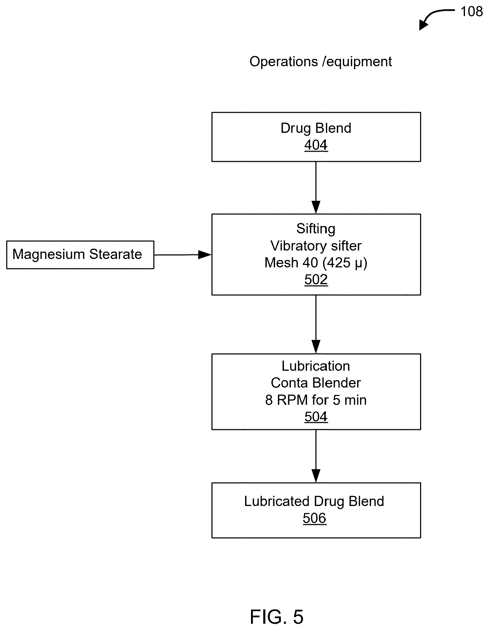

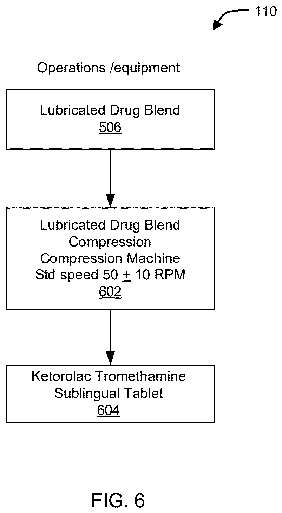

The process steps 102 of FIG. 1 are described in detail in FIG. 2. The process steps 104 of FIG. 1 are described in detail in FIG. 3. The process step 106 of FIG. 1 is described in detail in FIG. 4. The process step 108 of FIG. 1 is described in detail in FIG. 5. The process step 110 of FIG. 1 is described in detail in FIG. 6.

Again, referring to FIG. 1, at step 102, the process 100 involves dry mixing of ketorolac tromethamine, a diluent, and an antistatic agent to form dry mix blend. The process step 102 of FIG. 1 is described in detail in FIG. 2.

In one or more embodiments, in the step of the dry mixing, the diluent is lactose monohydrate, and the diluent is present in an amount of about 55 to about 60 percent by weight; and the antistatic agent is colloidal anhydrous silica, and the antistatic agent is present in an amount of about 0.2 to about 0.8 percent by weight.

As used herein, lactose monohydrate is a water-soluble filler and diluent with good compressibility. It helps in facilitating faster disintegration. It further contributes to improving the mouthfeel of a tablet. In an example, lactose monohydrate is Supertab 30 GR.

As used herein, colloidal anhydrous silica is an adsorbent, anticaking agent, and glidant. It enhances the flow of powder. In an example, colloidal anhydrous silica is Aerosil^{®} 200. Colloidal anhydrous silica is selected for the process of preparation to improve the flow property of the drug blend. Drug blend is elaborately discussed in the preceding paragraphs.

Referring to FIG. 2, at step 202, ketorolac tromethamine, lactose monohydrate, and colloidal anhydrous silica are co-sifted through 40 mesh of the vibratory sifter to obtain agglomerate free mass. The agglomerate free mass of the co-sifted ketorolac tromethamine, lactose monohydrate, and colloidal anhydrous silica is collected.

Again, referring to FIG. 2, at step 204, the agglomerate free mass of the co-sifted ketorolac tromethamine, lactose monohydrate, and colloidal anhydrous silica is loaded into a conta blender and mixed at the speed of 8 RPM for 30 minutes of mixing time. Resultantly, at step 206, dry mix blend is obtained.

At step 104, the process 100 involves intermediate blending a cushioning agent, a sweetener and a disintegrating agent to form an intermediate blend. The process step 104 of FIG. 1 is described in detail in FIG. 3.

In one or more embodiments, in the step of the intermediate blending, the cushioning agent is microcrystalline cellulose, and the cushioning agent is present in an amount of about 15 to about 20 percent by weight; the sweetener is sucralose, and the sweetener is present in an amount of about 3 to about 5 percent by weight; and the disintegrating agent is crospovidone, and the disintegrating agent is present in an amount of about 2 to about 7 percent by weight.

As used herein, microcrystalline cellulose is crystalline in nature. Microcrystalline cellulose enhances the flow of powder and helps in tablet compression to give desired hardness. It also acts as a cushioning agent. Due to the plastic nature of microcrystalline cellulose, it is often used as an excipient for both tablet compression and pelletizing processes. Microcrystalline cellulose as a cushioning agent fills the space between the particles and dampens the effect of the compressive force occurring through the tableting process. In an example, microcrystalline cellulose is Vivapur^{®} 102.

As used herein, sucralose is white to off-white colored, free flowing, crystalline powder which has sweetening power approximately 300-1000 times that of sucrose and has no after taste.

As used herein, crospovidone is a water insoluble tablet disintegrant or disintegrating agent and dissolution agent. Crospovidone rapidly exhibits high capillary activity and pronounced hydration capacity with little tendency to form gels. In an example, crospovidone is Kollidone^{®} CL. Crospovidone is selected for this tablet composition to improve the dispersion time of the tablet in the oral cavity.

Referring to FIG. 3, at step 302, microcrystalline cellulose, sucralose, crospovidone, are co-sifted through 40 mesh of the vibratory sifter to obtain agglomerate free mass of co-sifted material. At step 304, the co-sifted material obtained at step 302 is blended in the conta blender at 8 RPM for 10 minutes to obtain intermediate blend at step 306. Thereafter, at step 308, the 50% of the intermediate blend obtained at step 306 is unloaded (hereinafter, "50% of the unloaded intermediate blend") and the blender bin has 50% of the intermediate blend (hereinafter, "blender bin containing 50% of the intermediate blend"). Therefore, at step 310, the blender bin containing 50 % of the intermediate blend is obtained.

Again, referring to FIG. 1, at step 106, the process 100 involves drug blending the dry mix blend and the intermediate blend to form a drug blend. The process step 106 of FIG. 1 is described in detail in FIG. 4.

Referring to FIG. 4, the process step 402 comprises addition of dry mix blend obtained at step 206 of FIG. 2 to the blender bin containing 50% of the intermediate blend obtained at step 310 of the FIG. 3 to obtain a mixture. Further, adding the 50% of the unloaded intermediate blend obtained at step 308 to the mixture of the blender bin containing 50% of the intermediate blend and dry mix blend to obtain an added mixture. Thereafter, the added mixture is blended in a conta blender at the blending speed of 8 RPM for 30 minutes of blending time resulting in formation of drug blend at step 404.

Again, referring to FIG. 1, at step 108, the process 100 involves preparing a lubricated drug blend comprising sifting a lubricant and lubricating the drug blend with the lubricant to form a lubricated drug blend. The lubricant is magnesium stearate. The process step 108 of FIG. 1 is described in detail in FIG. 5.

In one or more embodiments, in the step of lubricating the drug blend, the lubricant is magnesium stearate, and the lubricant is present in an amount of about 1 to about 2 percent by weight.

As used herein, magnesium stearate is an impalpable powder with low bulk density. It is very fine and greasy in texture, light in weight, white in color, precipitated or milled in nature. Functionally, it is used as a lubricant in capsule and tablet manufacturing. Magnesium stearate powder, because of its characteristic greasiness adheres readily to the surface of tablet to ensure tablet is cleanly ejected without cracking or breakage in the pharmaceutical industry. Magnesium stearate is selected to prevent punching and die wall sticking during compression of the ketorolac tromethamine sublingual tablet. Compression is elaborately discussed in the preceding paragraphs.

Referring to FIG. 5, at step 502, the magnesium stearate is sifted through 40 mesh of the vibratory sifter to obtain agglomerate free mass of magnesium stearate. Thereafter, at step 504, the drug blend obtained at step 404 is lubricated with agglomerate free mass of magnesium stearate in the conta blender at the speed of 8 RPM for 5 minutes of mixing time. At step 506, the lubricated drug blend is obtained.

Again, referring to FIG. 1, at step 110, the process 100 involves compressing the lubricated drug blend to form a ketorolac tromethamine sublingual tablet. The process step 110 of FIG. 1 is described in detail in FIG. 6.

Referring to FIG. 6, at step 602, the lubricated drug blend obtained at step 506 is compressed in a compression machine at a standard speed of 50 ± 10 RPM to obtain ketorolac tromethamine sublingual tablet at step 604.

In one or more embodiments, the lactose monohydrate and microcrystalline cellulose are in a molar ratio of 3:1.

Lactose monohydrate in combination with microcrystalline cellulose is selected to have a free-flowing mass during tableting operation ultimately contributing towards quicker dispersion with better mouthfeel to the tablet composition.

Herein, the molar ratio of lactose monohydrate to microcrystalline cellulose is 3:1 in direct compression formulations for sublingual tablets. This specific ratio is primarily based on the balance between hardness, compressibility, and disintegration time of sublingual tablets.

In terms of compressibility, lactose monohydrate is a well-known filler and diluent with good compressibility, but it may not provide optimal tablet hardness when used alone in sublingual tablets. On the other hand, microcrystalline cellulose has excellent compressibility properties and helps in improving hardness of a tablet, which is crucial for maintaining structural integrity during manufacturing and handling.

In terms of enhancing mouthfeel, microcrystalline cellulose is slightly coarse in nature. Therefore, a higher proportion of lactose monohydrate helps in improving the mouthfeel of sublingual tablets.

In terms of disintegration, lactose monohydrate is a water-soluble excipient that helps in facilitating faster disintegration. Moreover, microcrystalline cellulose, though not soluble in water, assists in creating a porous tablet matrix that allows rapid water uptake and ultimately promotes disintegration. Molar ratio of lactose monohydrate to microcrystalline cellulose as 3:1 promotes disintegration of the sublingual tablet as the sublingual tablet must disintegrate rapidly in the mouth to ensure quick release of the active pharmaceutical ingredient (API), herein ketorolac tromethamine.

In terms of flowability, microcrystalline cellulose improves the overall flowability of the powder blend during direct compression, however, a high proportion of microcrystalline cellulose may affect the disintegration time. Therefore, the molar ratio as 3:1 balances the flow property with the desired disintegration speed.

In one or more embodiments, the particle size of lactose monohydrate is not more than 150 microns.

In TABLE 1, the test results for compliance with standard specification of particle size distribution (PSD) of the lactose monohydrate is depicted.

**TABLE 1**

| **Test** | **Specification** | **Observation** |
|---|---|---|
| Particle size distribution (PSD) | 10 % - 30 % particles should be less than 75 microns 40 % - 70 % particles should be less than 150 microns. | 18.02 % particles less than 75 microns and 49.97 % particles less than 150 microns. |

The specific particle size of lactose monohydrate contributes to minimizing the risk of unequal distribution of the ketorolac tromethamine in the tablet composition.

In one or more embodiments, the ketorolac tromethamine sublingual tablet is packed in a blister package.

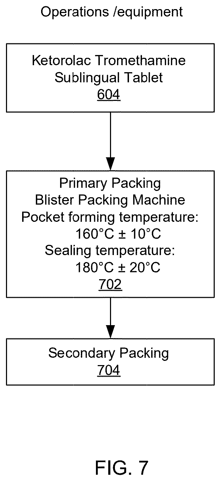

Referring to FIG. 7, the ketorolac tromethamine sublingual tablet obtained at step 604 is packed through a two-step packing process. The two steps are a primary packing process and a secondary packing process. At step 702, in the primary packing process, a blister packing machine is used with temperature parameters pocket forming temperature of 160°C ± 10°C; and sealing temperature of 180°C ± 20°C. Thereafter, the primary packed ketorolac tromethamine sublingual tablet is further packed through secondary packing process at step 704.

In one or more embodiments, the present disclosure relates to ketorolac tromethamine sublingual tablet or tablet composition. The ketorolac tromethamine sublingual tablet comprises about 10 to about 15 percent by weight of ketorolac tromethamine; about 55 to about 60 percent by weight of a diluent; about 0.2 to about 0.8 percent by weight of an antistatic agent; about 15 to about 20 percent by weight of a cushioning agent; about 3 to about 5 percent by weight of a sweetener; about 2 to about 7 percent by weight of a disintegrating agent; and about 1 to about 2 percent by weight of a lubricant.

Ketorolac tromethamine is tromethamine salt of ketorolac (C₁₅H₁₃NO₃. C₄H₁₁NO), with molecular formula C₁₉H₂₄N₂O₆ is also known by a chemical name "benzoyl-2,3-dihydro-1H-pyrrolizine-1-carboxylic acid". IUPAC name of the ketorolac tromethamine is "2-amino-2-(hydroxymethyl)propane-1,3-diol;5-benzoyl-2,3-dihydro-1H-pyrrolizine-1-carboxylic acid".

Ketorolac tromethamine is a synthetic pyrrolizine carboxylic acid derivative with anti-inflammatory, analgesic and antipyretic properties. Ketorolac tromethamine, a non-selective inhibitor of the cyclooxygenases (COX), inhibits both COX-1 and COX-2 enzymes. Ketorolac tromethamine as an active ingredient exhibits a strong bitter taste.

In one or more embodiments, the dosage amount of the ketorolac tromethamine sublingual tablet is 10 mg per tablet.

In one or more embodiments, the diluent is lactose monohydrate; the antistatic agent is colloidal anhydrous silica; the cushioning agent is microcrystalline cellulose; the sweetener is sucralose; the disintegrating agent is crospovidone; and the lubricant is magnesium stearate.

In one or more embodiments, the lactose monohydrate and microcrystalline cellulose are in a molar ratio of 3:1.

In one or more embodiments, the particle size of lactose monohydrate is not more than 150 microns.

In one or more embodiments, the friability is less than 1.5 %; the disintegration time is less than 300 seconds; and the hardness is from about 20 N to about 60 N.

In TABLE 2, the weight percentage of API and excipients for the exhibit batch is depicted.

**TABLE 2**

| **DRY MIXING** | | |
|---|---|---|
| **Ingredients** | **Function** | **Relative Qty. (% w/w)** |
| Ketorolac Tromethamine | Active | 12.5 |
| Lactose monohydrate (Supertab 30 GR) | Diluent | 57.75 |
| Silica, Colloidal Anhydrous (Aerosil 200) | Antistatic agent | 0.5 |
| | | |

| **INTERMEDIATE BLENDING** | | |
|---|---|---|
| Cellulose Microcrystalline (Vivapur 102) | Cushioning agent | 18.75 |
| Sucralose | Sweetener | 4.0 |
| Crospovidone (Kollidone^{®}CL) | Disintegrating Agent | 5.0 |

| **LUBRICATION** | | |
|---|---|---|
| Magnesium Stearate | Lubricant | 1.5 |
| Total | | 100.0 |

### EXAMPLE 1

In Example 1, a process for preparing ketorolac tromethamine sublingual tablet is described. Particularly, the process is described for preparing ketorolac tromethamine sublingual tablet having a strength of 10 mg per tablet.

In TABLE 3, the amount of API and excipients (in mg/tablet) for ketorolac tromethamine (10 mg) sublingual tablet are depicted.

**TABLE 3**

| **DRY MIXING** | |
|---|---|
| Ingredients | Relative Qty. (in mg/tablet) |
| Ketorolac Tromethamine | 10.0 mg |
| Lactose monohydrate (Supertab 30 GR) | 46.2 mg |
| Colloidal Anhydrous Silica (Aerosil 200) | 0.4 mg |

| **INTERMEDIATE BLENDING** | |
|---|---|
| Cellulose Microcrystalline (Vivapur 102) | 15.0 mg |
| Sucralose | 3.2 mg |
| Crospovidone (Kollidone^{®}CL) | 4.0 mg |

| **LUBRICATION** | |
|---|---|
| Magnesium Stearate | 1.2 mg |
| Total | 80.0 mg |

The process is initiated by preparation a dry mix blend by dry mixing 10 mg ketorolac tromethamine, 46.2 mg diluent, and 0.4 mg antistatic agent. Thereafter, an intermediate blend is prepared by an intermediate blending 15 mg cushioning agent, 3.2 mg sweetener and 4.0 mg disintegrating agent. Thereafter, a drug blend is prepared by blending the dry mix blend, and the intermediate blend is prepared in the above steps. Thereafter, a lubricated drug blend is prepared by initially sifting 1.2 mg lubricant followed by lubricating the drug blend with the lubricant. Finally, a ketorolac tromethamine sublingual tablet is prepared by compressing the lubricated drug blend.

Specifically, 10 mg of ketorolac tromethamine, 46.2 mg of lactose monohydrate, and 0.4 mg of colloidal anhydrous silica are co-sifted through 40 mesh of the vibratory sifter to obtain agglomerate free mass. The agglomerate free mass of the co-sifted ketorolac tromethamine, lactose monohydrate, and colloidal anhydrous silica is collected.

Thereafter, the agglomerate free mass of the co-sifted ketorolac tromethamine, lactose monohydrate, and colloidal anhydrous silica is loaded into a conta blender and mixed at the speed of 8 RPM from 30 minutes of mixing time resulting in preparation of a dry mix blend.

Subsequently, 15 mg of microcrystalline cellulose, 3.2 mg of sucralose, and 4.0 mg of crospovidone, are co-sifted through 40 mesh of the vibratory sifter to obtain agglomerate free mass of co-sifted material. The co-sifted material obtained is blended in the conta blender at 8 RPM for 10 minutes resulting in the formation of intermediate blend.

Following which, the 50 % of the intermediate blend is unloaded as "50% of the unloaded intermediate blend" and the rest of the 50 % of the intermediate blend remained in the blender bin of the conta blender as "blender bin containing 50 % of the intermediate blend".

Thereafter, a previously prepared dry mix blend is added to the blender bin containing 50% of the intermediate blend to obtain a mixture. The mixture is further added with 50% of the unloaded intermediate blend resulting in preparation of an added mixture. Thereafter, the added mixture is blended in a conta blender at the blending speed of 8 RPM for 30 minutes of blending time resulting in formation of drug blend.

Subsequently, 1.2 mg magnesium stearate is sifted through 40 mesh of the vibratory sifter to obtain agglomerate free mass of magnesium stearate. Thereafter, the previously prepared drug blend is lubricated with agglomerate free mass of magnesium stearate in the conta blender at the speed of 8 RPM for 5 minutes of mixing time resulting in preparation of lubricated drug blend.

Finally, the lubricated drug blend is compressed through a compression machine at a standard speed of 50 ± 10 RPM resulting in formation of ketorolac tromethamine sublingual tablet. This composition is the finalized tablet composition for ketorolac tromethamine sublingual tablet to achieve improved palatability and quick dispersion of the active drug.

In TABLE 4, the analytical results for the finalized tablet composition for ketorolac tromethamine sublingual tablet is depicted.

**TABLE 4**

| **Parameters (units)** | **Observations** |
|---|---|
| Tablet Weight (mg) | 80 |
| Punch size (mm) | 6 mm |
| Surface of the tablet | Round flat |
| Hardness (N) | 34 N - 46 N |
| Dispersion time (seconds) | 214 seconds |
| Taste | Good taste, acceptable mouth feel |
| Conclusion | Composition is not sticking. Satisfactory taste and dispersion time |

The finalized tablet composition for ketorolac tromethamine sublingual tablet is put for the development trail for blending and compression purposes in two different batches.

In TABLE 5A and 5B, the analytical results of 10 mg two batches of ketorolac tromethamine sublingual tablet are depicted; wherein TABLE 5A and 5B, depict results for BATCH 1 and BATCH 2 respectively.

**TABLE 5A**

| **Parameters (units)** | **Observations** |
|---|---|
| Tablet Weight (mg) | 80 |
| Punch size (mm) | 6 mm |
| Surface of the tablet | Round flat |
| Hardness (N) | 36 N - 48 N |
| Dispersion time (seconds) | 226 seconds |
| Taste | Enhanced taste, acceptable mouth feel |
| Conclusion | Preferable process with satisfactory test parameters |

**TABLE 5B**

| **Parameters (units)** | **Observations** |
|---|---|
| Tablet Weight (mg) | 80 |
| Punch size (mm) | 6 mm |
| Surface of the tablet | Round flat |
| Hardness (N) | 36 N - 46 N |
| Dispersion time (seconds) | 253 seconds |
| Taste | Enhanced taste, acceptable mouth feel |
| Conclusion | Preferable process with satisfactory test parameters |

### Tests:

To compare the bioavailability and characterize the pharmacokinetic profile of the prepared ketorolac tromethamine 10 mg sublingual tablet (hereinafter, "test product") relative to that of reference product is conducted to assess the bioequivalence of the test product. This test is further objected to monitor the adverse events and to ensure the safety of the consumers.

### Crossover Bioequivalence Study

An open label, balanced, randomized, two-treatment, two-sequence, two-period, single oral dose, crossover bioequivalence study of test product and the reference product in normal, healthy, adult, human male and female volunteers under fasting conditions is performed.

In TABLE 6, the result of the crossover bioequivalence study is depicted.

**TABLE 6**

| **Parameters** | **Geometric Least Squares Means** | | | **90% Confidence Interval** | **Intra subject CV (%)** | **Inter subject CV (%)** | **Power (%)** |
|---|---|---|---|---|---|---|---|
| | **Test Product-T (N = 37)** | **Reference Product-R (N = 37)** | **T/R Ratio (%)** | | | | |
| ln Cₘₐₓ | 1066.632 | 1047.435 | 101.8 | 97.49 - 106.37 | 11.1 | 19.5 | 100.0 |
| ln AUC₀₋ₜ | 4439.149 | 4371.096 | 101.6 | 98.29 - 104.93 | 8.3 | 24.9 | 100.0 |
| ln AUC_{0-∞} | 4653.211 | 4599.349 | 101.2 | 97.78 - 104.68 | 8.7 | 26.6 | 100.0 |

The test observations reveal that the test product in comparison with the reference product meets the bioequivalence criteria with respect to Cmax and AUC₀₋ₜ for ketorolac tromethamine under fasting conditions as per criteria set in the protocol.

Conclusively, the test product has improved pharmacokinetic profile as compared to the same dose of reference product administered by oral pharmaceutical composition. Herein, the improved pharmacokinetic profile means increase in Cmax or AUC₀₋ₜ of ketorolac tromethamine.

Mean plasma concentration versus nominal time curve for ketorolac tromethamine with reference to the test product and the reference product is plotted to determine the bioequivalence of the test product and the reference product.

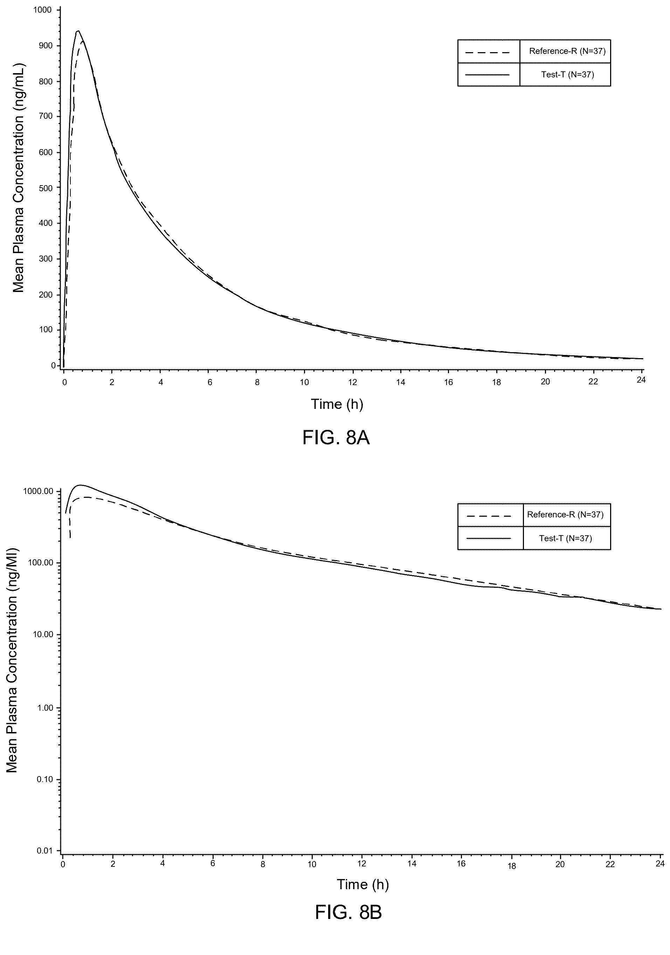

Referring to FIG. 8A, the linear plot for the mean plasma concentration on one axis (Y-axis) versus time curve on the other (X-axis) for the ketorolac tromethamine of the test product and the reference product is plotted.

The analysis of the graphical curves of the linear plot reveals enhanced bioequivalence for the test product in comparison with the bioequivalence of the reference product.

Referring to FIG. 8B, the semilogarithmic plot for the mean plasma concentration on one axis (Y-axis) versus time curve on the other (X-axis) for the ketorolac tromethamine of the test product and the reference product is plotted.

The analysis of the graphical curves of the semilogarithmic plot confirms enhanced bioequivalence for the test product in comparison with the bioequivalence of the reference product.

The sublingual tablet prepared by the present disclosure as sublingual administration of ketorolac tromethamine refers to the placement of drug under the tongue. The sublingual route bypasses the first-pass metabolism and hence facilitates rapid absorption of the drug into the systemic circulation. Drug directly reaches the systemic circulation using blood vessels. The sublingual region holds a rich source of blood vessels which are routed parallel to the mucosal surface. The sublingual artery supplies blood to the salivary glands. This way the wastage of drug herein ketorolac tromethamine is minimized.

As used herein, the active ingredient, ketorolac tromethamine exhibits a strong bitter taste and therefore an efficient taste-masking is opted to improve the palatability. Specifically, the diluent, the antistatic agent, the cushioning agent, the sweetener, the disintegrating agent, and the lubricant (hereinafter, collectively referred to as "pharmaceutical excipients") are used within the specific range of percentage by weight for contributing into a tablet composition for sublingual or buccal administration to achieve quick dispersion with good mouth feel for the sublingual tablet.

The selection of the pharmaceutical excipients is for the main objective of tablet matrix formulation with good organoleptic properties with positive effect on taste masking and mouth sensation; quick disintegration time in mouth; suitable flowability of drug blend; and convenience during tableting. Moreover, the selection of pharmaceutical excipients exhibits no punch or die wall sticking during compression.

Herein, tablet composition consists of excipients with similar particle size for minimizing the risk of unequal distribution of the ketorolac tromethamine.

The foregoing descriptions of specific embodiments of the present disclosure have been presented for purposes of illustration and description. They are not intended to be exhaustive or to limit the present disclosure to the precise forms disclosed, and obviously many modifications and variations are possible in light of the above teaching. The embodiments were chosen and described in order to best explain the principles of the present disclosure and its practical application, to thereby enable others skilled in the art to best utilize the present disclosure and various embodiments with various modifications as are suited to the particular use contemplated. (It is understood that various omissions and substitutions of equivalents are contemplated as circumstance may suggest or render expedient, but such are intended to cover the application or implementation without departing from the spirit or scope of the claims of the present disclosure).

## Claims

1. A process for preparation of ketorolac tromethamine sublingual tablet comprising:
dry mixing ketorolac tromethamine, a diluent, and an antistatic agent to form a dry mix blend;
intermediate blending a cushioning agent, a sweetener and a disintegrating agent to form an intermediate blend;
drug blending the dry mix blend and the intermediate blend to form a drug blend;
preparing a lubricated drug blend comprising
sifting a lubricant,
lubricating the drug blend with the lubricant to form a lubricated drug blend; and
compressing the lubricated drug blend to form a ketorolac tromethamine sublingual tablet.

2. The process as claimed in claim 1, wherein in the step of the dry mixing:
the diluent is lactose monohydrate, and the diluent is present in an amount of about 55 to about 60 percent by weight; and
the antistatic agent is colloidal anhydrous silica, and the antistatic agent is present in an amount of about 0.2 to about 0.8 percent by weight.

3. The process as claimed in claim 1, wherein in the step of the intermediate blending:
the cushioning agent is microcrystalline cellulose, and the cushioning agent is present in an amount of about 15 to about 20 percent by weight;
the sweetener is sucralose, and the sweetener is present in an amount of about 3 to about 5 percent by weight; and
the disintegrating agent is crospovidone, and the disintegrating agent is present in an amount of about 2 to about 7 percent by weight.

4. The process as claimed in claim 1, wherein in the step of lubricating the drug blend:
the lubricant is magnesium stearate, and the lubricant is present in an amount of about 1 to about 2 percent by weight.

5. The process as claimed in claims 2 and 3, wherein the lactose monohydrate and microcrystalline cellulose are in a molar ratio of 3:1.

6. The process as claimed in claim 2, wherein the particle size of lactose monohydrate is not more than 150 microns.

7. The process as claimed in claim 1, wherein the ketorolac tromethamine sublingual tablet is packed in a blister package.

8. A ketorolac tromethamine sublingual tablet, the ketorolac tromethamine sublingual tablet comprising:
about 10 to about 15 percent by weight of ketorolac tromethamine;
about 55 to about 60 percent by weight of a diluent;
about 0.2 to about 0.8 percent by weight of an antistatic agent;
about 15 to about 20 percent by weight of a cushioning agent;
about 3 to about 5 percent by weight of a sweetener;
about 2 to about 7 percent by weight of a disintegrating agent; and
about 1 to about 2 percent by weight of a lubricant.

9. The ketorolac tromethamine sublingual tablet as claimed in claim 8, wherein the dosage amount of the ketorolac tromethamine sublingual tablet is 10 mg per tablet.

10. The ketorolac tromethamine sublingual tablet as claimed in claim 8, wherein the diluent is lactose monohydrate; the antistatic agent is colloidal anhydrous silica; the cushioning agent is microcrystalline cellulose; the sweetener is sucralose; the disintegrating agent is crospovidone; and the lubricant is magnesium stearate.

11. The ketorolac tromethamine sublingual tablet as claimed in claim 10, wherein the lactose monohydrate and microcrystalline cellulose are in a molar ratio of 3:1.

12. The ketorolac tromethamine sublingual tablet as claimed in claim 10, wherein the particle size of lactose monohydrate is not more than 150 microns.

13. The ketorolac tromethamine sublingual tablet as claimed in claim 8, wherein the friability is less than 1.5 %; the disintegration time is less than 300 seconds; and the hardness is from about 20 N to about 60 N.
